# EUROPEAN PATENT APPLICATION

(11) **EP 4 509 049 A1**
(43) Date of publication of application: **19.02.2025**
(21) Application number: 22943910.4
(22) Date of filing: 25.08.2022
(51) Int. Cl.: A61B 5/157, A61B 5/151, A61B 5/15, A61B 5/145, A61B 5/021, A61B 5/1455, A61B 5/00

(54) **BLOOD COLLECTION AND BLOOD GLUCOSE MEASUREMENT DEVICE**

(30) Priority: 25.05.2022 KR 20220064350
(71) Applicant: Lameditech Co., Ltd., Seoul 08513 (KR)
(72) Inventor: CHOI, Jong Seok, Incheon 21376 (KR); CHO, Jae Yeon, Buyeo-gun Chungcheongnam-do 33111 (KR); HWANG, Seok Hwan, Seoul 08849 (KR); JEON, Yun Ha, Incheon 22716 (KR)
(74) Representative: Manitz Finsterwald Patent- und Rechtsanwaltspartnerschaft mbB
(86) International application number: PCT/KR2022/012718
(87) International publication number: WO 2023/229103

(57) **Abstract**

A lancing and blood glucose level measuring device includes a casing; a laser oscillation unit that is accommodated within the casing and configured to generate a laser beam for blood sampling; a blood glucose level measuring unit that is accommodated within the casing and configured to measure a blood glucose level; a contact unit that is formed on a part of one side of the casing and configured to irradiate the laser beam toward an irradiation target area; a blood glucose strip insertion unit which is formed on another part of one side of the casing and into which a blood glucose strip including a blood glucose sensor that transmits and receives signals to and from the blood glucose level measuring unit to measure a blood glucose level is to be inserted; and a controller configured to control the laser oscillation unit.

## Description

### TECHNICAL FIELD

The present disclosure relates to a lancing and blood glucose level measuring device.

### BACKGROUND

Recently, as medical devices have been developed, laser devices are increasingly applied in diagnosis and treatment of various diseases. Such a medical laser device is configured to perforate skin by irradiating a laser beam to the skin in order to sample blood, administer a drug into the perforated skin, or remove moles or freckles from the skin.

Such a medical laser device needs to secure safety in use because it is configured to irradiate a laser beam directly to the skin. For example, the laser device needs to be accurately located at a site to be perforated and needs to stay at the same site during irradiation of the laser beam. Also, it is important for a user to stably grip the device when the laser beam is irradiated.

In this regard, Patent Document 1 (Korean Patent Laid-open Publication No. 10-2018-0096404 A (published on August 29, 2018)) discloses a cap for collecting blood and a laser lancing device having the cap. Specifically, in Patent Document 1, the cap to be attached to and detached from the laser lancing device is located between the laser lancing device and the skin to be perforated, and the cap is disposable to prevent infection.

However, it is not easy for the conventional laser lancing device to transport collected blood to a blood sampling sensor in order to measure a blood glucose level from the collected blood.

### DISCLOSURE OF THE INVENTION

### PROBLEMS TO BE SOLVED BY THE INVENTION

The present disclosure provides a lancing and blood glucose level measuring device in which a laser oscillation unit and a contact unit are provided and the laser oscillation unit generates a laser beam for blood sampling and the contact unit irradiates the laser beam toward an irradiation target area to perform blood sampling.

The present disclosure also provides a lancing and blood glucose level measuring device in which a blood glucose level measuring unit and a blood glucose strip insertion unit are provided and the blood glucose strip insertion unit enables a blood glucose strip including a blood glucose sensor to be inserted and the blood glucose level measuring unit measures a blood glucose level.

However, problems to be solved by the present disclosure are not limited to the above-described problems. There may be other problems to be solved by the present disclosure.

### MEANS FOR SOLVING THE PROBLEMS

As a means for solving the problems, according to an aspect of the present disclosure, a lancing and blood glucose level measuring device includes a casing; a laser oscillation unit that is accommodated within the casing and configured to generate a laser beam for blood sampling; a blood glucose level measuring unit that is accommodated within the casing and configured to measure a blood glucose level; a contact unit that is formed on a part of one side of the casing and configured to irradiate the laser beam toward an irradiation target area; a blood glucose strip insertion unit which is formed on another part of one side of the casing and into which a blood glucose strip including a blood glucose sensor that transmits and receives signals to and from the blood glucose level measuring unit to measure a blood glucose level is to be inserted; and a controller configured to control the laser oscillation unit.

According to the present disclosure, the blood glucose strip includes a blood absorbing member formed of a super absorbent polymer to absorb blood remaining and exposed at a peripheral portion of the blood glucose sensor. The super absorbent polymer is spaced apart from the blood glucose sensor by a predetermined distance.

According to the present disclosure, the lancing and blood glucose level measuring device further includes a heating member that is formed on yet another part of one side of the casing and configured to apply heat around the irradiation target area.

According to the present disclosure, the blood glucose level measuring unit includes an optical measuring sensor that measures at least one of a blood glucose level, a blood pressure and a blood oxygen level.

According to the present disclosure, the casing further includes a communication module that transmits blood glucose level data measured by the blood glucose level measuring unit to a user device connected to the lancing and blood glucose level measuring device.

According to the present disclosure the contact unit includes a disposable cap to be in contact with the irradiation target area.

According to the present disclosure, the contact unit further includes a pressure sensor that senses a pressure generated by contact between the irradiation target area and the disposable cap.

According to the present disclosure, when a pressure value sensed by the pressure sensor is equal to or higher than a predetermined value, the controller operates the laser oscillation unit.

The above-described aspects are provided by way of illustration only and should not be construed as liming the present disclosure. Besides the above-described embodiments, there may be additional embodiments described in the accompanying drawings and the detailed description.

### EFFECTS OF THE INVENTION

According to any one of the aspects of the present disclosure, it is possible to provide a lancing and blood glucose level measuring device in which a contact unit irradiates a laser beam to an irradiation target area to sample blood and a blood glucose level measuring unit measures a blood glucose level from the sampled blood. Thus, it is possible to sample blood and measure a blood glucose level using a single device .

Also, according to the present disclosure, it is possible to provide a lancing and blood glucose level measuring device in which a contact unit coupled to a disposable cap and a blood glucose strip insertion unit coupled to a blood glucose strip that are formed on the same connecting structure enable sampled blood to be smoothly transported to a blood glucose sensor.

### BRIEF DESCRIPTION OF THE DRAWINGS

**FIG. 1A** is a diagram illustrating the appearance of a lancing and blood glucose level measuring device in accordance with an embodiment of the present disclosure.
**FIG. 1B** is a diagram illustrating the appearance of a lancing and blood glucose level measuring device in accordance with an embodiment of the present disclosure.
**FIG. 1C** is a diagram illustrating the appearance of a lancing and blood glucose level measuring device in accordance with an embodiment of the present disclosure.
**FIG. 2** illustrates internal components of the lancing and blood glucose level measuring device in accordance with an embodiment of the present disclosure.
**FIG. 3** is a diagram for explaining a process of sampling blood and measuring a blood glucose level by using the lancing and blood glucose level measuring device in accordance with an embodiment of the present disclosure.
**FIG. 4A** is a diagram for explaining a blood glucose strip shown in **FIG. 3****.**
**FIG. 4B** is a diagram for explaining a blood glucose strip shown in **FIG. 3****.**
**FIG. 5** is a diagram for explaining a heating member of the lancing and blood glucose level measuring device in accordance with an embodiment of the present disclosure.
**FIG. 6** is a diagram for explaining a blood glucose level measuring unit including an optical measuring sensor of the lancing and blood glucose level measuring device in accordance with an embodiment of the present disclosure.

### BEST MODE FOR CARRYING OUT THE INVENTION

Hereafter, embodiments of the present disclosure will be described in detail with reference to the accompanying drawings so that the present disclosure may be readily implemented by a person with ordinary skill in the art. However, it is to be noted that the present disclosure is not limited to the embodiments but may be embodied in various other ways. In drawings, parts irrelevant to the description are omitted for the simplicity of explanation, and like reference numerals denote like parts through the whole document.

Through the whole document, the term "connected to" or "coupled to" that is used to designate a connection or coupling of one element to another element includes both a case that an element is "directly connected or coupled to" another element and a case that an element is "electronically connected or coupled to" another element via still another element. Further, through the whole document, the term "comprises or includes" and/or "comprising or including" used in the document means that one or more other components, steps, operation and/or existence or addition of elements are not excluded in addition to the described components, steps, operation and/or elements unless context dictates otherwise.

Through the whole document, the term "unit" includes a unit implemented by hardware, a unit implemented by software, and a unit implemented by both of them. One unit may be implemented by two or more pieces of hardware, and two or more units may be implemented by one piece of hardware.

Through the whole document, a part of an operation or function described as being carried out by a terminal or device may be carried out by a server connected to the terminal or device. Likewise, a part of an operation or function described as being carried out by a server may be carried out by a terminal or device connected to the server.

Hereinafter, an embodiment of the present disclosure will be described in detail with reference to the accompanying drawings.

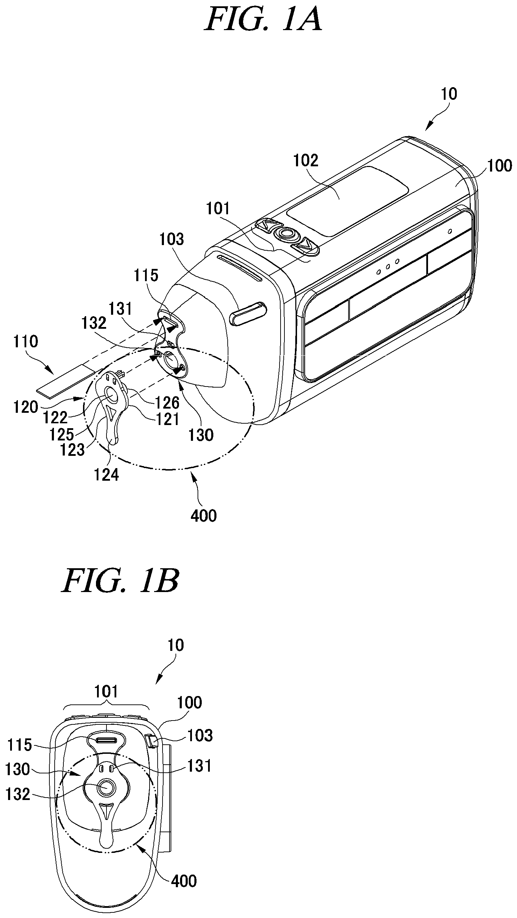

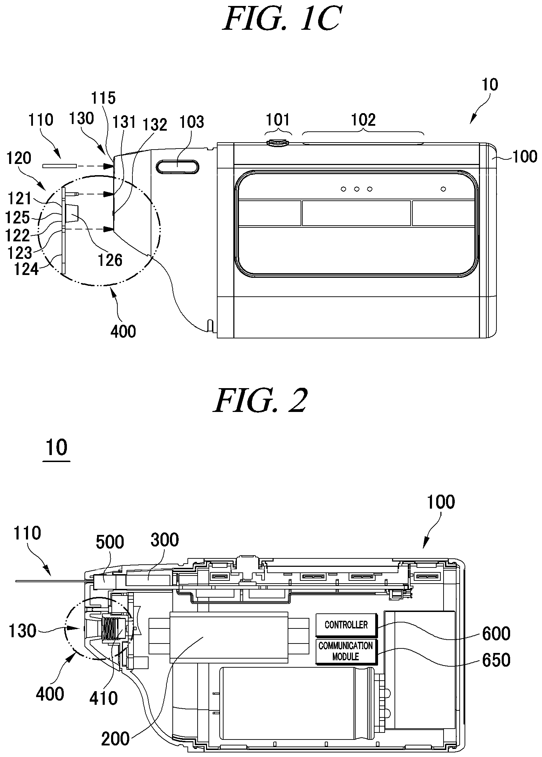

**FIG. 1A** to **FIG. 1C** are diagrams illustrating the appearance of a lancing and blood glucose level measuring device in accordance with an embodiment of the present disclosure. Specifically, **FIG. 1A** is a perspective view of a lancing and blood glucose level measuring device 10, **FIG. 1B** is a front view of the lancing and blood glucose level measuring device 10, and **FIG. 1C** is a side view of the lancing and blood glucose level measuring device 10.

Referring to **FIG. 1A** to **FIG. 1C****,** the lancing and blood glucose level measuring device 10 may include a casing 100, a plurality of buttons 101, a display part 102, a strip eject button 103, a blood glucose strip connector 115 and a contact unit 400.

The casing 100 forms the appearance of the lancing and blood glucose level measuring device 10, and a component for irradiating a laser beam to an irradiation target area may be located within the casing 100. The casing 100 may have a shape easy for a user to grip by hand.

The plurality of buttons 101 may include buttons for a power switch and an intensity regulator. The power switch may turn on and off a power supply that serves as an energy source of laser. The intensity regulator may regulate the intensity of a laser beam oscillated from a laser oscillation unit 200.

The display part 102 may display a blood glucose level measured from sampled blood. Also, the display part 102 may display the intensity regulated by the intensity regulator.

The plurality of buttons 101 may be configured as physical buttons or a touch panel for the power switch and the intensity regulator, and the display part 102 may be a liquid crystal display. However, the present disclosure is not limited thereto. The power switch, the intensity regulator and the display part may be displayed on a single touch display, and when the user touches the touch display, the intensity of power or a laser beam may be regulated.

When a blood glucose strip 110 is inserted into the blood glucose strip connector 115, the strip eject button 103 may allow the blood glucose strip 110 to be ejected from the blood glucose strip connector 115. The strip eject button 103 may be configured as a physical button or a touch panel. For example, when the user applies a predetermined pressure to the strip eject button 103, the strip eject button 103 may allow the blood glucose strip 110 inserted into the blood glucose strip connector 115 may be automatically ejected. Accordingly, since the strip eject button 103 allows the blood glucose strip 110 to be ejected from the blood glucose strip connector 115, the user does not need to remove the blood glucose strip 110 stained with blood by hand. Thus, it is possible to suppress contamination caused by the blood glucose strip 110.

The blood glucose strip 110 including a blood glucose sensor may be inserted into the blood glucose strip connector 115. After the measurement of the blood glucose level, the blood the blood glucose strip 110 may be ejected from the lancing and blood glucose level measuring device 10 by the user.

The blood glucose strip 110 may include therein the blood glucose sensor. The blood glucose strip 110 is a means used to analyze blood glucose from blood and may output an electrical current corresponding to a blood glucose level of supplied blood. The blood glucose strip 110 may be a consumable product that is replaced each time a blood glucose level is measured. The blood glucose strip 110 may include an electrode unit configured to measure a blood glucose level, and a reagent may be coated on the electrode unit. The electrode unit may include a working electrode and a reference electrode.

The contact unit 400 may be formed at a part of one side of the casing 100. For example, the contact unit 400 may include a disposable cap 120 to be in contact with the irradiation target area and a disposable cap connector 130 for coupling between the disposable cap 120 and the casing 100 at a part of one side of the casing 100.

The disposable cap 120 may be coupled to the disposable cap connector 130 formed at a main body of the casing 100 for blood sampling. For example, the disposable cap 120 may be replaceably coupled to the disposable cap connector 130 located in front of a beam barrel 410 (see FIG. 2). This is to facilitate coupling or separation between the disposable cap 120 and the disposable cap connector 130 for replacement of disposable cap 120. Thus, after the existing disposable cap is separated from the disposable cap connector 130, a fresh disposable cap is coupled to the disposable cap connector 130. Therefore, it is possible to suppress cross-contamination between patients during blood sampling. The disposable cap 120 is configured to be brought into contact with skin and may minimize smell, smoke and noise which may be generated during perforation of the skin. Particularly, the disposable cap 120 can block direct contact between the beam barrel 410 and the skin and thus can suppress contamination of the beam barrel. Further, each user can replace the disposable cap 120, and, thus, it is possible to suppress cross-contamination between patients who use the lancing and blood glucose level measuring device 10 together.

The disposable cap 120 may include a cap main body 121, an opening 122 and a support surface 123 formed along the circumference of the opening 122 so as to be supported on a contact area. For example, the cap main body 121 may be formed into a circular plate shape and may include the opening 122 in an axial direction in the middle thereof. Further, the cap main body 121 may include at least one grip portion 124 extending from the support surface 123.

The user may easily detach or attach the disposable cap 120 from or to the lancing and blood glucose level measuring device 10 by using the grip portion 124. The opening 122 may have an area such that a laser beam output from the lancing and blood glucose level measuring device 10 is not interrupted by a through-hole 125 of the disposable cap 120 and a traveling path of the laser beam can be secured.

Also, the disposable cap 120 may be designed to be smaller than the average area of a fingertip with which the disposable cap 120 is to be in contact, and, thus, an appropriate pressure may be applied to the irradiation target area. Likewise, the support surface 123 may have an appropriate area such that the disposable cap 120 can press against a contact area with which the disposable cap 120 is in contact.

Further, the disposable cap 120 may include a clamping unit 126 coupled to the cap main body 121. The clamping unit 126 may couple the cap main body 121 to the beam barrel 410 via the disposable cap connector 130. For example, the clamping unit 126 is located at a peripheral portion on a back surface of the disposable cap 120 and protrudes backwards, and may include a protrusion formed on an inner surface. Furthermore, the protrusion may be engaged with a clamping groove 131 formed in the disposable cap connector 130 so as to suppress any unintentional separation of the disposable cap 120.

The disposable cap connector 130 may be coupled to the disposable cap 120 for blood sampling. For example, when the disposable cap connector 130 is coupled to the disposable cap 120, the disposable cap connector 130 may allow a laser beam generated by the laser oscillation unit 200 to pass through the beam barrel 410 and the laser beam passing through the beam barrel 410 to be irradiated to the irradiation target area through the disposable cap 120 coupled to the disposable cap connector 130.

The disposable cap connector 130 may include the clamping groove 131 and a laser irradiation unit 132. For example, the disposable cap connector 130 may be coupled to the disposable cap 120 via the clamping groove 131. Herein, when the disposable cap 120 is coupled to the disposable cap connector 130 via the clamping groove 131, the disposable cap connector 130 may allow a laser beam generated by the laser oscillation unit 200 to be irradiated by the laser irradiation unit 132.

The contact unit 400 may irradiate a laser beam toward the irradiation target area. For example, the contact unit 400 includes internal components composed of, for example, at least one measuring sensor configured to sense a contact with the irradiation target area, a contact sensing circuit connected to the measuring sensor and the barrel 410 through which a laser beam passes, within the casing 100. A laser beam generated by the laser oscillation unit 200 can be irradiated toward the irradiation target area by the internal components included in the contact unit 400. The internal components included in the contact unit 400 will be described below with reference to FIG. **2****.**

**FIG. 2** illustrates internal components of the lancing and blood glucose level measuring device in accordance with an embodiment of the present disclosure. Referring to FIG. 1 and FIG. **2****,** the lancing and blood glucose level measuring device 10 may include the casing 100, the laser oscillation unit 200, a blood glucose level measuring unit 300, the contact unit 400, a blood glucose strip insertion unit 500, a controller 600 and a communication module 650.

The laser oscillation unit 200 is accommodated within the casing 100 and configured to generate a laser beam for blood sampling. For example, the laser oscillation unit 200 is supplied with electrical energy and amplifies light to generate optical energy such as laser selected from gas laser, liquid laser and solid laser and then emits the optical energy outwards and forwards.

The blood glucose level measuring unit 300 is accommodated within the casing 100 and configured to measure a blood glucose level.

For example, the blood glucose level measuring unit 300 may include a circuit module that senses an electrical signal through the blood glucose strip 110 and converts the sensed electrical signal into a blood glucose level.

The blood glucose level measuring unit 300 may measure a blood glucose level by using amperometry. Amperometry is a technique in which oxidation and reduction of a material are conducted using an electrode, followed by conversion into electrical current. An electrode active material such as an enzyme involved in oxygen reaction or microbial response may react with a power supply and may be output as an electrical current value. By measuring the electrical current value, it is possible to measure the concentration of substrate such as glucose, ascorbic acid, etc. Thus, amperometry is mainly used for various biosensors. Measurement methods using amperometry may include an equilibrium method (normal method) of measuring a normal electrical current proportional to concentration and a speed method using the relationship between a change in speed of electrical current and concentration. For example, the blood glucose level measuring unit 300 may measure a blood glucose level by using an electrical current generated when a constant voltage is applied to the blood glucose strip 110 for a predetermined period of time. When a constant voltage is applied to the blood glucose strip 110 for a predetermined period of time, an electrical current is generated in proportion to a blood glucose level. When the electrical current value is applied to a relation formula between a blood glucose level and an electrical current, the blood glucose level can be measured.

For another example, the blood glucose level measuring unit 300 further includes an optical measuring sensor, and may further include a circuit module that senses an optical signal through the optical measuring sensor and converts the sensed optical signal into an electrical signal.

The blood glucose level measuring unit 300 may use the optical measuring sensor to measure a blood glucose level by a non-invasive method or blood sampling method. For example, the optical measuring sensor outputs light having a wavelength of 300 nm to 3,000 nm and a photo diode (PD) converts absorbed light or reflected light into an electrical current value, and then, a blood glucose level is expressed based on the measured value. For example, the blood glucose level measuring unit 300 may use the optical measuring sensor to generate light and irradiate the light to blood and may analyze a change in intensity of reflected light over time by optical reflectometry, and, thus, a blood glucose level can be measured based on the result of analysis.

For yet another example, the blood glucose level measuring unit 300 may use the optical measuring sensor to irradiate light having at least one wavelength to blood, calculate the absorbance (degree of light absorption by a specific line segment) from reflected light or transmitted light and measure the ingredients of blood based on the absorption spectrum.

The contact unit 400 includes the disposable cap 120 and the disposable cap connector 130 outside the casing 100, and may include at least one measuring sensor for sensing a contact with the irradiation target area and a contact sensing circuit connected to the at least one measuring sensor inside the casing 100. Herein, the disposable cap 120 and the disposable cap connector 130 have been described above with reference to FIG. 1A to FIG. 1C and thus will not be described in detail.

The contact unit 400 may include the beam barrel 410 accommodated within the casing 100 and configured to irradiate a laser beam.

The beam barrel 410 is accommodated within the casing 100 and provides a path through which a laser beam irradiated by the laser oscillation unit 200 passes to the skin. To this end, the lancing and blood glucose level measuring device 10 may further include a forward and backward moving means (not shown) to move the beam barrel 410 forwards and backwards. In other words, the forward and backward moving means moves the beam barrel 410 forwards or backwards to adjust the distance between the user's skin and a focusing lens and thus adjust the size of a laser spot irradiated to the irradiation target area of the user. For example, as the user adjusts the size of a laser spot using the plurality of buttons 101, the motor is driven and the position of the beam barrel 410 is adjusted. However, the present disclosure is not limited thereto. The position of the beam barrel 410 may be manually moved forwards or backwards.

The contact unit 400 may include a pressure sensor that senses a pressure generated by contact between the irradiation target area and the disposable cap 120. Herein, when a pressure value sensed by the pressure sensor is equal to or higher than a predetermined value, the laser oscillation unit 200 may operate under control of the controller 600.

The contact unit 400 may be in contact with the at least one measuring sensor provided within the casing 100.

Herein, the at least one measuring sensor may include, for example, a sensor for the disposable cap 120, a sensor for the blood glucose strip 110, a contact sensor configured to sense a contact with the irradiation target area and a pressure sensor configured to measure a pressure applied to the irradiation target area in order to sense whether the lancing and blood glucose level measuring device 10 operates safely.

For example, the lancing and blood glucose level measuring device 10 can simultaneously perform contact sensing and pressure sensing through a micro array lens (MLA) and a pogo pin of the contact unit 400 or a conductor that can sense an electrical current.

For example, contact sensors on both sides of an aperture of the lancing and blood glucose level measuring device 10 and a pressure sensor configured to measure a specific pressure (for example, a voltage of 0.1 V or more or an electrical current of 0.1 µAh or more) may be used to simultaneously perform contact sensing and pressure sensing.

For example, the pogo pin of the lancing and blood glucose level measuring device 10 may sense a pressure when a predetermined pressure is applied, and the contact sensing circuit may be connected through the pogo pin by the applied pressure.

The sensor for the disposable cap 120 may sense whether the disposable cap 120 is reused or replaced. Herein, the sensor for the disposable cap 120 may be a photo sensor.

For example, when the disposable cap 120 is reused, a surface through which a laser beam passes becomes opaque. Thus, when the disposable cap 120 is reused, blood sampling cannot be performed. Therefore, the sensor for the disposable cap 120 can sense whether the disposable cap 120 is reused when the surface reflectance of a transparent window through which a laser beam passes is lower than a specific value. That is, when the sensor for the disposable cap 120 determines that the surface reflectance of the transparent window is lower than a specific value, an alarm indicating that the disposable cap 120 has been reused may be output through the display part 102 or by sound.

Alternatively, the sensor for the disposable cap 120 may sense whether the disposable cap 120 is a new cap or a replaced one by using a pressure or push-pull switch. For example, the pressure switch is located where a cap is to be inserted at a front end of the aperture, and when the disposable cap 120 is initially inserted into the lancing and blood glucose level measuring device 10, the pressure switch is pressed, and, thus, the sensor for the disposable cap 120 recognizes that the disposable cap 120 is a new disposable cap. Also, when the disposable cap 120 is removed after a laser beam is oscillated and when the pressure switch is released over several micro-time periods, the sensor for the disposable cap 120 may sense replacement with a new disposable cap. If there is no such a signal, the lancing and blood glucose level measuring device 10 may output an alarm so that the user can replace the disposable cap 120.

A blood glucose strip includes therein a plurality of circuit lines, and, thus, the sensor for the blood glucose strip 110 can measure a resistance value of circuit lines. For example, when the sensor for the blood glucose strip 110 senses a resistance value of a specific circuit pattern after the blood glucose strip 110 is inserted into the blood glucose strip connector 115, the sensor for the blood glucose strip 110 may sense insertion of the blood glucose strip 110.

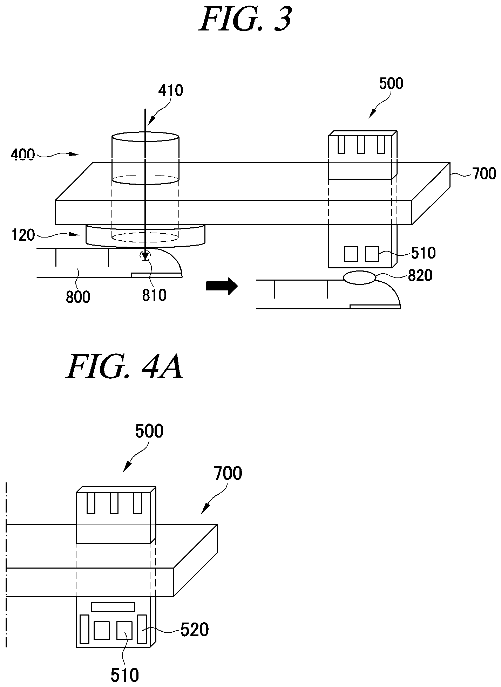

The blood glucose strip insertion unit 500 may be formed at another part of one side of the casing 100, and the blood glucose strip 110 including a blood glucose sensor 510 (see FIG. 3) that transmits and receives signals to and from the blood glucose level measuring unit 300 to measure a blood glucose level can be inserted into blood glucose strip insertion unit 500. For example, the blood glucose strip 110 may be inserted into the blood glucose strip connector 115 of the blood glucose strip insertion unit 500, and the blood glucose strip 110 may be ejected after the measurement of the blood glucose level.

The blood glucose strip insertion unit 500 can perform insertion and ejection of the blood glucose strip 110 and can also serve as an interface between the blood glucose strip 110 and the lancing and blood glucose level measuring device 10. To serve as an interface, the blood glucose strip insertion unit 500 may include a terminal that applies a voltage to an electrode unit of the blood glucose strip 110 and a terminal that detects an electrical current received from the electrode unit. These terminals may enable the blood glucose strip insertion unit 500 to serve as an interface and may also enable the blood glucose strip insertion unit 500 to provide an elastic force for ejecting the blood glucose strip 110.

For example, the blood glucose strip insertion unit 500 may check whether the blood glucose strip 110 has been inserted into the blood glucose strip connector 115. When the blood glucose strip 110 is inserted into the blood glucose strip connector 115, a predetermined electrical current flows between the lancing and blood glucose level measuring device 10 and the blood glucose strip 110. Accordingly, the controller 600 can sense whether the blood glucose strip 110 has been inserted.

The controller 600 can control the laser oscillation unit 200. For example, the controller 600 can operate the laser oscillation unit 200 when the user applies a signal, the pressure sensor included in the contact unit 400 senses a pressure generated by contact between the irradiation target area and the disposable cap and the pressure value sensed by the pressure sensor is equal to or higher than a predetermined value.

The controller 600 may regulate the power or the intensity of a laser beam based on signals input from the plurality of buttons 101. For example, when an on/off control signal is input from the power switch, the controller 600 may turn on/off the power supply that serves as an energy source of laser. For another example, when a control signal involved in regulating the intensity of a laser beam is input from the button for the intensity regulator, the controller 600 may regulate forward and backward movement of the beam barrel 410 based on the regulated intensity of a laser beam.

When the contact unit 400 coupled to the disposable cap 120 is in contact with at least one measuring sensor, the controller 600 may derive the amount of change in electrical current of the irradiation target area through the at least one measuring sensor in contact with the contact unit 400 coupled to the disposable cap 120 and may control the operation of the laser oscillation unit 200 based on the derived amount of change in electrical current. Herein, the blood glucose strip or a part of the disposable cap 120 and the contact unit 400 is formed of a metal and is in contact with the sensor of the lancing and blood glucose level measuring device 10. Thus, the timing of laser oscillation can be determined based on a change in amount of electric current caused by contact with skin.

For example, when a skin contact sensor in contact with the contact unit 400 senses a contact with skin for a predetermined period of time, the controller 600 may control the laser oscillation unit 200 to oscillate a laser beam.

The communication module 650 is formed within the casing 100 and may transmit blood glucose level data measured by the blood glucose level measuring unit 300 to a user device connected to the lancing and blood glucose level measuring device 10. Herein, the blood glucose level data may be displayed through a blood glucose information application installed on the user device, and various kinds of information depending on a blood glucose level may be recommended through the blood glucose information application.

**FIG. 3** is a diagram for explaining a process of sampling blood and measuring a blood glucose level by using the lancing and blood glucose level measuring device in accordance with an embodiment of the present disclosure. Referring to **FIG. 3****,** the lancing and blood glucose level measuring device 10 may irradiate a laser beam to the user's skin, perform blood sampling and then measure a blood glucose level from the sampled blood of the user through the blood glucose strip 110.

For example, when the disposable cap 120 coupled to the contact unit 400 is in contact with the user's skin 800, the laser oscillation unit 200 may generate a laser beam for blood sampling. Herein, the contact unit 400 may irradiate the laser beam generated by the laser oscillation unit 200 toward the user's skin 800 through the beam barrel 410. As a result, a perforation is formed in a portion 810 of the user's skin 800 and the user's blood is exposed.

Then, if blood is sampled from the user's skin 800, the user may move his/her finger toward the blood glucose strip 110, and, thus, the user's blood 820 exposed for blood sampling may be brought into contact with the blood glucose strip 110 including the blood glucose sensor 510 coupled to the blood glucose strip insertion unit 500. Herein, a blood glucose level of the user can be measured by the blood glucose level measuring unit 300.

According to an embodiment of the present disclosure, the contact unit 400 and the blood glucose strip insertion unit 500 of the lancing and blood glucose level measuring device 10 may be formed on the same connecting structure 700.

Therefore, after blood sampling, the user may smoothly move the irradiation target area with blood to the blood glucose strip 110. Accordingly, it is possible to easily measure a blood glucose level from the blood.

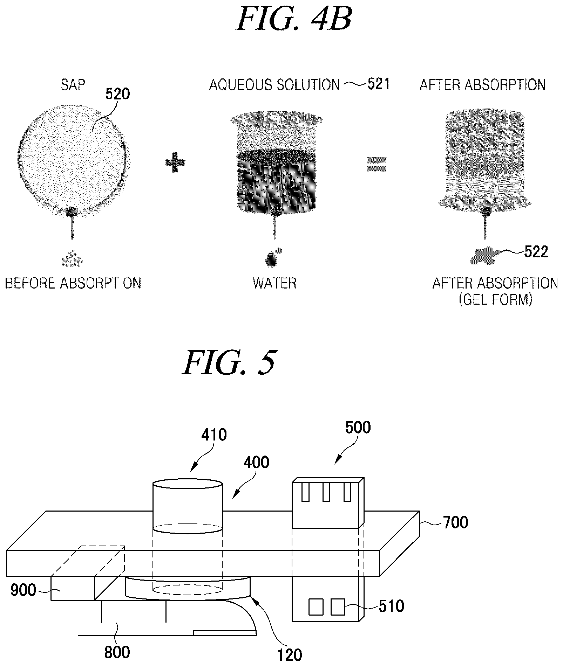

**FIG. 4A** and **FIG. 4B** are diagrams for explaining a blood glucose strip shown in **FIG. 3****.**

Referring to **FIG. 4A****,** the blood glucose strip 110 may include a blood absorbing member 520 formed of a super absorbent polymer to absorb blood remaining and exposed at a peripheral portion of the blood glucose sensor 510. Herein, the super absorbent polymer may be spaced apart from the blood glucose sensor 510 by a predetermined distance (for example, 0.5 mm or more).

When a conventional blood measuring device measures a blood glucose level using a blood glucose strip, the strip with blood is removed after the measurement of the blood glucose level, but in this case, any remaining blood may splatter. Therefore, the blood measuring device may be contaminated.

However, according to the present disclosure, the super absorbent polymer is spaced apart from the blood glucose sensor 510 by a predetermined distance at the peripheral portion of the blood glucose sensor 510 of the blood glucose strip 110. Therefore, after the measurement of the blood glucose level, the super absorbent polymer may absorb blood remaining on a surface of the blood glucose sensor 510 and thus suppress external contamination.

**FIG. 4B** illustrates the performance of the blood absorbing member formed of the super absorbent polymer.

In general, blood is made up of blood cells (about 45%) and plasma (about 55%), and water accounts for about 91% of plasma.

When blood is absorbed using the super absorbent polymer, the super absorbent polymer shows high ability to absorb blood and convert the absorbed blood into gel form.

Therefore, if the blood absorbing member 520 formed of the super absorbent polymer is located at the peripheral portion of the blood glucose sensor 510, the blood absorbing member 520 can sufficiently absorb remaining blood after the measurement of the blood glucose level. Thus, it is possible to suppress contamination of the lancing and blood glucose level measuring device 10 by blood during replacement of the blood glucose strip 110.

**FIG. 5** is a diagram for explaining a heating member of the lancing and blood glucose level measuring device in accordance with an embodiment of the present disclosure.

Referring to **FIG. 5****,** the lancing and blood glucose level measuring device 10 may further include a heating member 900 that is formed on yet another part of one side of the casing 100 and applies heat around the irradiation target area.

For example, when the user's finger skin 800 is brought into contact with the disposable cap 120, the heating member 900 applies heat around the irradiation target area to increase a flow rate of blood within the user's skin. Accordingly, since the flow rate of blood within the user's skin is increased by applying heat to the user's skin, the effectiveness of blood sampling can be improved.

The heating member 900 may be formed to be located at a lower end of the blood glucose strip 110. When the user brings his/her finger or skin into contact with the disposable cap 120 for blood sampling, the heating member 900 may apply heat to a temperature at which the user does not feel unpleasant due to the heat and thus may increase a flow rate of blood within the user's skin.

Conventionally, when a blood glucose level is measured from blood sampled from the user, a negative pressure is used to increase a flow rate of blood. However, when the negative pressure is used, a negative pressure pump is needed to transport blood to a blood sampling sensor provided within a blood glucose level measuring device or the blood glucose level measuring device needs to be processed complicatedly.

However, according to the present disclosure, it is possible to easily measure a blood glucose level without using a negative pressure.

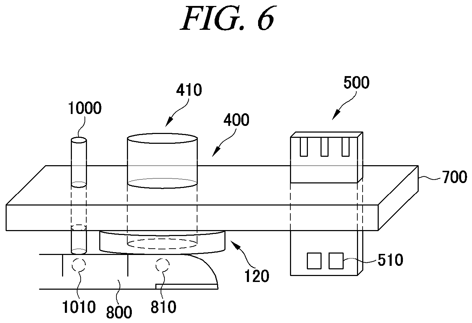

**FIG.** 6 is a diagram for explaining a blood glucose level measuring unit including an optical measuring sensor of the lancing and blood glucose level measuring device in accordance with an embodiment of the present disclosure.

Referring to **FIG. 6****,** the blood glucose level measuring unit 300 may further include an optical measuring sensor 1000 that measures at least one of a blood glucose level, a blood pressure and a blood oxygen level. Herein, the optical measuring sensor 1000 may be formed on yet another part of one side of the casing 100 of the lancing and blood glucose level measuring device 10.

For example, when the user's finger skin 800 is brought into contact with the disposable cap 120, the optical measuring sensor 1000 may optically measure a blood glucose level, a blood pressure or a blood oxygen level from the inside of the skin 800 or sampled blood by a non-invasive method or blood sampling method. Herein, the blood glucose level measured by the optical measuring sensor 1000 is compared with a blood glucose level actually measured by blood sampling, and, thus, the accuracy in measuring a blood glucose level by the lancing and blood glucose level measuring device 10 can be improved.

The above description of the present disclosure is provided for the purpose of illustration, and it would be understood by a person with ordinary skill in the art that various changes and modifications may be made without changing technical conception and essential features of the present disclosure. Thus, it is clear that the above-described embodiments are illustrative in all aspects and do not limit the present disclosure. For example, each component described to be of a single type can be implemented in a distributed manner. Likewise, components described to be distributed can be implemented in a combined manner.

The scope of the present disclosure is defined by the following claims rather than by the detailed description of the embodiment. It shall be understood that all modifications and embodiments conceived from the meaning and scope of the claims and their equivalents are included in the scope of the present disclosure.

## Claims

1. A lancing and blood glucose level measuring device, comprising:
a casing;
a laser oscillation unit that is accommodated within the casing and configured to generate a laser beam for blood sampling;
a blood glucose level measuring unit that is accommodated within the casing and configured to measure a blood glucose level;
a contact unit that is formed on a part of one side of the casing and configured to irradiate the laser beam toward an irradiation target area;
a blood glucose strip insertion unit which is formed on another part of one side of the casing and into which a blood glucose strip including a blood glucose sensor that transmits and receives signals to and from the blood glucose level measuring unit to measure a blood glucose level is to be inserted; and
a controller configured to control the laser oscillation unit.

2. The lancing and blood glucose level measuring device of Claim 1,
wherein the blood glucose strip includes a blood absorbing member formed of a super absorbent polymer to absorb blood remaining and exposed at a peripheral portion of the blood glucose sensor.

3. The lancing and blood glucose level measuring device of Claim 2,
wherein the super absorbent polymer is spaced apart from the blood glucose sensor by a predetermined distance.

4. The lancing and blood glucose level measuring device of Claim 1, further comprising:
a heating member that is formed on yet another part of one side of the casing and configured to apply heat around the irradiation target area.

5. The lancing and blood glucose level measuring device of Claim 1,
wherein the blood glucose level measuring unit includes an optical measuring sensor that measures at least one of a blood glucose level, a blood pressure and a blood oxygen level.

6. The lancing and blood glucose level measuring device of Claim 1,
wherein the casing further includes a communication module that transmits blood glucose level data measured by the blood glucose level measuring unit to a user device connected to the lancing and blood glucose level measuring device.

7. The lancing and blood glucose level measuring device of Claim 1,
wherein the contact unit includes a disposable cap to be in contact with the irradiation target area.

8. The lancing and blood glucose level measuring device of Claim 7,
wherein the contact unit further includes a pressure sensor that senses a pressure generated by contact between the irradiation target area and the disposable cap.

9. The lancing and blood glucose level measuring device of Claim 8,
wherein when a pressure value sensed by the pressure sensor is equal to or higher than a predetermined value, the controller operates the laser oscillation unit.
